# EUROPEAN PATENT SPECIFICATION

(11) **EP 4 268 858 B1**
(45) Date of publication and mention of the grant of the patent: **17.12.2025**
(21) Application number: 22836607.6
(22) Date of filing: 16.05.2022
(51) Int. Cl.: A61L 27/50, A61L 27/36, A61L 2/00

(54) **BIOLOGICAL TISSUE MATERIAL AND PREPARATION METHOD THEREFOR**
BIOLOGISCHES GEWEBEMATERIAL UND HERSTELLUNGSVERFAHREN DAFÜR
MATÉRIAU TISSULAIRE BIOLOGIQUE ET SON PROCÉDÉ DE PRÉPARATION

(30) Priority: 09.11.2021 CN 202111322401
(43) Date of publication of application: 01.11.2023
(73) Proprietor: Jiangsu Trulive Medtech Co., Ltd, Rudong County Nantong Jiangsu 226400 (CN)
(72) Inventor: YANG, Wei, Nantong, Jiangsu 226400 (CN); ZHAO, Jing, Nantong, Jiangsu 226400 (CN)
(74) Representative: Pohlman, Sandra M.
(86) International application number: PCT/CN2022/092949
(87) International publication number: WO 2023/279851

(56) References cited:
- WO-A1-2018/153525
- CN-A- 104 998 299
- CN-A- 109 651 627
- CN-A- 111 701 079
- CN-A- 112 773 936
- CN-A- 114 209 886
- KR-A- 20160 028 253
- US-A- 6 008 292
- US-A1- 2007 269 478
- US-A1- 2020 171 205
- US-B2- 8 357 387

## Description

### TECHNICAL FIELD

The present invention relates to the field of biotechnology and, in particular, to a biological tissue material and a method of preparing the material.

### BACKGROUND

Biological tissues commonly used in the contemporary clinical practice (e.g., animal-derived collagenous fibrous tissue materials for use in biological valves, biological patches, etc.) are usually cross-linked and fixed with chemical reagents (e.g., by being stored in glutaraldehyde and/or formaldehyde). Biological tissues are often stored in dilute aqueous solutions containing glutaraldehyde and/or formaldehyde, which provide sterile environments where components of the biological tissues can remain hydrated. However, a large number of studies have shown that residual glutaraldehyde in such biological tissue prostheses that have been implanted will promote valvular calcification. Moreover, as glutaraldehyde itself is highly toxic, even a minor amount of glutaraldehyde that remains will produce toxicity in the human body, which is not favorable to endothelialization. For these reasons, a biological tissue prosthesis that has been treated and preserved with glutaraldehyde must undergo repeated extensive washing to remove glutaraldehyde before it can be implanted. However, prior to the implantation of biological tissue materials or biological tissue prostheses made thereof, it is desired that medical personnel minimizes preparatory work that may cause exposure of related medical devices, because "easily available, immediately usable" biological tissues and biological tissue prostheses can not only reduce the chance of contamination or errors, but can also shorten the time required for the implantation of a biological tissue prosthesis.

The above problems can be effectively mitigated by providing dry biological materials preserved in absence of glutaraldehyde. Decellularized biological tissue materials exhibit low immunogenicity, fibrous structural integrity and desirable mechanical strength. Decellularization is commonly accomplished physically or by enzymatic digestion, an acid or base, a chemical detergent, etc. However, a large number of studies have shown that these approaches are associated with many problems, such as emergence of immune responses due to insufficient decellularization or collagenous structural destruction caused by excessive decellularization, as well as, calcification possibly caused by adsorption of lipoproteins and calcium-binding proteins by phospholipids on the surface of implanted biological materials, which may in turn lead to hardening or degradation of the biological materials.

Therefore, developing an anti-immune, anti-calcification dry biological tissue material and a method of preparing such a material would promise a prospect of extensive future development and be of significant value to practical applications.

US 8 357 387 describes a method of mitigating calcification in bioprosthetic implant tissue comprising a) at least partially crosslinking bioprosthetic implant tissue using glutaraldehyde or other aldehyde containing agents; b) treating bioprosthetic implant tissue with a capping agent that reacts with and replaces functional groups on said tissue, and c) dehydrating the tissue with a non-aqueous solution.

### SUMMARY OF THE INVENTION

It is an object of the present invention to provide such an anti-immune, anti-calcification dry biological tissue material and a preparation method.

Accordingly, the present invention provides a method of preparing a dry biological tissue material as defined in claim 1, and which comprises the steps of:
step 1: obtaining a biological tissue material that has undergone a cross-linking treatment using a cross-linking agent;
step 2: performing a decellularization treatment on the biological tissue material using decellularization solutions, wherein the decellularization solutions include first to N-th decellularization solutions with gradually increasing concentrations, and wherein N is a positive integer that is greater than or equal to 2, followed by washing the biological tissue material that has undergone decellularization treatment;
step 3: performing a first anti-calcification treatment on the biological tissue material using a dissolving solution, wherein the dissolving solution is used to dissolve phospholipid impurities in the biological tissue;
step 4: performing a second anti-calcification treatment on the biological tissue material using a removal solution, wherein the removal solution is used to remove carboxyl groups produced in the cross-linking and decellularization treatments as a result of oxidation of aldehyde groups;
step 5: performing a third anti-calcification treatment on the biological tissue material using a reducing solution, wherein the reducing solution is used to protect free aldehyde groups; and
step 6: performing a dehydration treatment on the biological tissue material using dehydrating solutions to obtain the dry biological tissue material, wherein the dehydrating solutions include first to M-th dehydrating solutions with gradually increasing concentrations, and wherein the concentration of the M-th dehydrating solution is lower than the concentration of the N-th decellularization solution, and M is a positive integer that is greater than or equal to 2.

In this preparation method the cross-linking agent is an aldehyde-based cross-linking agent comprising glutaraldehyde, formaldehyde and glyoxal.

In step 2 the decellularization solution is an aqueous solution of a monoalcohol or a polyol;
optionally step 2 may further comprise placing the biological tissue material in the first to N-th decellularization solutions for at least 1 hour in each solution, so as to perform the decellularization treatment on the biological tissue material. Decellularization treatment is followed by a step of
washing the biological tissue material that has undergone the decellularization treatment.

Additionally, sequentially placing the biological tissue material in the first to N-th decellularization solutions for at least 1 hour in each solution may comprise:
when the decellularization solutions include a first decellularization solution with a concentration of 50-70% and a second decellularization solution with a concentration of 70-100%, placing the biological tissue material in the first decellularization solution for at least 1 hour and then in the second decellularization solution for at least 1 hour; or
when the decellularization solutions include a first decellularization solution with a concentration of 50-60%, a second decellularization solution with a concentration of 60-80% and a third decellularization solution with a concentration of 80-100%, placing the biological tissue material in the first decellularization solution for at least 1 hour, then in the second decellularization solution for at least 1 hour and finally in the third decellularization solution for at least 1 hour.

The dissolving solution used in step 3 has a concentration of 1-100%, wherein the dissolving solution comprises at least one of methanol, ethanol, chloroform and diethyl ether and has a pH value of 5 0-9.0. Step 3 may further comprise the steps of
placing the biological tissue material in the dissolving solution at 0°C -50 °C for 1-72 hours, so as to perform the first anti-calcification treatment on the biological tissue material; and
washing the biological tissue material that has undergone the first anti-calcification treatment.

The removal solution used in step 4 has a concentration of 0.01-50%, wherein the removal solution comprises at least one of sodium carbonate, sodium bicarbonate, 1,1,2-trimethyl-propyl-borane, diisobutylaluminum hydride, borane and sodium borohydride and has a pH value of 5 0-9.0. Step 4 may further include
placing the biological tissue material in the removal solution at -100°C -50 °C for 1-72 hours, so as to perform the second anti-calcification treatment; and
washing the biological tissue material that has undergone the second anti-calcification treatment.

The reducing solution used in step 5 has a concentration of 0.01-50%, wherein the reducing solution comprises a solution of at least one of metaphosphate, amino acid, 3-dimethylaminopropyl and amino alcohol and has a pH value of 5 0-9.0. Step 5 may further include
placing the biological tissue material in the reducing solution at 0°C -50 °C for 1-72 hours, so as to perform the third anti-calcification treatment on the biological tissue material; and
washing the biological tissue material that has undergone the third anti-calcification treatment.

The dehydrating solutions used in step 6 are solutions of one or more of acetone, ethanol, n-butanol and tert-butanol. Step 6 may further comprise sequentially placing the biological tissue material in the first to M-th dehydrating solutions at 0°C -50 °C for 1 -120 hours in each solution, so as to perform the dehydration treatment on the biological tissue material.

Optionally, the method may further comprise after step 6:
anaerobically packaging and sterilizing the dry biological tissue material, wherein the sterilization is accomplished by ethylene oxide (EO) or by radiation.

Optionally, the dry biological tissue material may be used to produce a dry valve.

The present invention has at least the following benefits over the prior art:
1. The three anti-calcification treatments respectively for dissolving phospholipids, removing carboxylic acids and protecting aldehyde groups make up a complete anti-calcification process, thereby achieving a high anti-calcification performance.
2. The anaerobic packaging and sterilization of the dry biological tissue material can additionally reduce the risk of calcification of the biological tissue material. Moreover, as the concentration of the M-th dehydrating solution is lower than that of the N-th decellularization solution, collagenous structural integrity can be ensured. The first to N-th decellularization solutions with gradually increased concentrations can effectively remove immunotoxic substances.
3. The dry biological tissue material prepared in accordance with the present invention has been well decellularized and exhibits low immune activation, good integrity and good mechanical properties. The biological tissue material in a dried state allows easy storage, transportation and use and has a reduced chance of contamination or errors.

### BRIEF DESCRIPTION OF THE DRAWINGS

Fig. 1 shows presence of calcium in bovine pericardium samples obtained according to Embodiment of the present application and Comparative Examples 1 to 5 that have been individually implanted in rats.
Fig. 2 shows a HE-stained bovine pericardium sample according to Embodiment of the present application.
Fig. 3 shows a HE-stained bovine pericardium sample according to Comparative Example 7.

### DETAILED DESCRIPTION

Embodiments of the present invention will be described below by way of specific examples. Based on the disclosure and teachings provided herein, a person of ordinary skill in the art will readily realize other advantages and benefits provided by the present invention.

In embodiments of the present invention, there is provided a method of preparing a dry (or dried) biological tissue material as defined in claim 1, and which comprises the steps of:
step 1: obtaining a biological tissue material that has undergone a cross-linking treatment using a cross-linking agent;
step 2: subjecting the biological tissue material to a decellularization treatment using decellularization solutions, wherein the decellularization solutions include first to N-th decellularization solutions with gradually increasing concentrations, where N is a positive integer that is greater than or equal to 2, followed by a step of washing the biological tissue material that has undergone decellularization treatment;
step 3: subjecting the biological tissue material to a first anti-calcification treatment using a dissolving solution for dissolving phospholipid impurities in the biological tissue;
step 4: subjecting the biological tissue material to a second anti-calcification treatment using a removal solution for removing carboxyl groups produced in the cross-linking and decellularization processes as a result of oxidation of aldehyde groups;
step 5: subjecting the biological tissue material to a third anti-calcification treatment using a reducing solution for protecting free aldehyde groups; and
step 6: subjecting the biological tissue material to a dehydration treatment using dehydrating solutions to obtain the dry biological tissue material, wherein the dehydrating solutions include first to M-th dehydrating solutions with gradually increasing concentrations, and wherein the concentration of the M-th dehydrating solution is lower than the concentration of the N-th decellularization solution, and M is a positive integer that is greater than or equal to 2.

In particular, step 1 includes obtaining a biological tissue material that has undergone a cross-linking treatment using a cross-linking agent; and may further include subjecting the biological tissue material to a first washing process. The biological tissue material that has undergone the cross-linking treatment using the cross-linking agent may be heterogenic or allogeneic. Examples of the material may include pericardia, heart valves, peritonea, pleurae, small intestinal submucosae, dura mater, ligaments and skins. Particular examples may include bovine pericardia. The cross-linking agent is glutaraldehyde, formaldehyde or glyoxal. In the first washing process, a saline solution, a phosphate buffer with a pH value of 6.8-8.6 (in particular, for example, 6.8, 7.0, 7.2, 7.4, 7.6, 7.8, 8.0, 8.2, 8.4 or 8.6) or a D-Hanks solution with a pH value of 6.8-8.6 (in particular, for example, 6.8, 7.0, 7.2, 7.4, 7.6, 7.8, 8.0, 8.2, 8.4 or 8.6) may be used to wash the material 2 to 3 times at room temperature, 8-10 (in particular, for example, 8, 9 or 10 minutes ) minutes each time.

In this step, free aldehyde groups may be present in the biological tissue material that has undergone the cross-linking treatment using glutaraldehyde as the cross-linking agent.

In particular, step 2 includes subjecting the biological tissue material to a decellularization treatment using decellularization solutions, wherein the decellularization solutions include first to N-th decellularization solutions with gradually increasing concentrations, where N is a positive integer that is greater than or equal to 2; and subjecting the biological tissue material to a second washing process.

Specifically, at first, the decellularization solutions may be prepared for dissolving and removing cellular and intercellular substances from the biological tissue. The decellularization solutions are aqueous solutions of a monoalcohol or a polyol, in particular, formaldehyde, ethanol, ethylene glycol or the like. The decellularization solutions have at least two different concentrations. In particular, the decellularization solutions include first to N-th decellularization solutions with gradually increasing concentrations, where N is a positive integer that is greater than or equal to 2. The concentrations of the decellularization solutions may range from 50% to 100%. For example, when the decellularization solutions are two solutions with different concentrations (i.e., N=2), the first decellularization solution may have a concentration of 50%-70% (in particular, for example, 50%, 55%, 60%, 65% or 69.9%), and the second decellularization solution may have a concentration of 70%-100% (in particular, for example, 70%, 75%, 80%, 85%, 90%, 95% or 100%). As another example, when the decellularization solutions are three solutions with different concentrations (i.e., N=3), the first decellularization solution may have a concentration of 50%-60% (in particular, for example, 50%, 55% or 59.9%), the second decellularization solution may have a concentration of 60%-80% (in particular, for example, 60.1%, 65%, 70%, 75% or 79.9%), and the third decellularization solution may have a concentration of 80%-100% (in particular, for example, 80.1%, 85%, 90%, 95% or 100%).

The biological tissue material may be then successively placed in the first to N-th decellularization solutions for at least 1 hour in each solution. Continuing the example of two solutions with different concentrations, the biological tissue material may be first placed in the first decellularization solution having a concentration of 50%-70% for at least 1 hour and then in the second decellularization solution having a concentration of 70%-100% for at least 1 hour. Continuing the example of three solutions with different concentrations, the biological tissue material may be first placed in the first decellularization solution having a concentration of 50%-60% for at least 1 hour, then in the second decellularization solution having a concentration of 60%-80% for at least 1 hour, and finally in the third decellularization solution having a concentration of 80%-100% for at least 1 hour. In this step, the decellularization solutions with different concentrations (the permeating agent) can permeate into the biological tissue material to remove immune substances therefrom while allowing the biological tissue to desirably retain its structure, avoiding the destruction of collagen fibers due to excessive decellularization.

In the subsequent second washing process, the biological tissue material that has undergone decellularization treatment is washed with a mixed solution of 1-10 mM (in particular, for example, 1 mM, 2 mM, 5 mM, 7 mM, 9 mM or 10 mM) Tris-HCl with a pH value of 6.8-8.6 (in particular, for example, 6.8, 7.0, 7.2, 7.4, 7.6, 7.8, 8.0, 8.2, 8.4 or 8.6) and 1-5 mM (in particular, for example, 1 mM, 2 mM, 3 mM, 4 mM or 5 mM) EDTA at a temperature of 0-10 °C (in particular, for example, 0 °C, 1 °C, 3 °C, 5 °C, 7 °C, 9 °C or 10 °C) for 1-24 hours (in particular, for example, 1 hour, 5 hours, 10 hours, 12 hours, 15 hours, 20 hours, 22 hours or 24 hours); and then further treated with an SDS solution with a concentration of 0.5-2% (in particular, for example, 0.5%, 1%, 1.5% or 2%) for 1-24 hours (in particular, for example, 1 hour, 5 hours, 10 hours, 12 hours, 15 hours, 20 hours, 22 hours or 24 hours). The mixed solution of Tris-HCl and EDTA can desirably dissolve DNA, and the SDS solution with a concentration of 0.5-2% can satisfactorily dissolve intercellular substances and nuclear membranes in the biological tissue material.

In step 3 the biological tissue material is subjected to a first anti-calcification treatment using the dissolving solution for dissolving phospholipid impurities in the biological tissue material.

Specifically, the dissolving solution may be prepared with a concentration of 1-100% (in particular, for example, 1%, 10%, 20%, 30%, 40%, 50%, 60%, 70%, 80%, 90% or 100%). The dissolving solution is a solution of at least one of methanol, ethanol, chloroform and diethyl ether and has a pH value of 5.0-9.0 (in particular, for example, 5.0, 5.5, 6.0, 6.5, 7.0, 7.5, 8.0, 8.5 or 9.0).

In this step the biological tissue material may be placed in the dissolving solution at a temperature of 0-50 °C (in particular, for example, 0 °C, 10 °C, 20 °C, 30 °C, 40 °C or 50 °C) for 1-72 hours (in particular, for example, 1 hours, 12 hours, 24 hours, 36 hours, 48 hours, 60 hours or 72 hours) in order to allow phospholipid impurities on the surface of the biological tissue material to be dissolved therein, so as to reduce the binding of the phospholipid with calcium.

At last, a third washing process may be carried out with the same parameters as the second washing process.

Free aldehyde groups produced in step 1 can be readily oxidized in steps 2 and 3 into carboxylic acids, which are prone to binding with calcium ions, leading to calcification of the biological tissue material. In order to remove carboxyl groups of such carboxylic acids, a removal solution is prepared in step 4 and used to apply a second anti-calcification treatment to the biological tissue material. In this way, the carboxyl groups resulting from oxidation of the aldehyde groups in the cross-linking and decellularization processes can be removed, resulting in a reduced number of calcium-binding sites. In this step, the removal is a solution of at least one of sodium carbonate, sodium bicarbonate, 1,1,2-trimethyl-propyl-borane, diisobutylaluminum hydride, borane and sodium borohydride.

Specifically, at first, the removal solution may be prepared with a concentration of 0.01-50% (in particular, for example, 0.01%, 1%, 10%, 20%, 30%, 40% or 50%) and a pH value of 5.0-9.0 (in particular, for example, 5.0, 5.5, 6.0, 6.5, 7.0, 7.5, 8.0, 8.5 or 9.0).

Next, the biological tissue material may be placed in the removal solution at a temperature of -100-50 °C (in particular, for example, -100 °C, -80 °C, -60 °C, -50 °C, -20 °C, 0 °C, 10 °C, 20 °C, 30 °C, 40 °C or 50 °C) for 1-72 hours (in particular, for example, 1 hour, 12 hours, 24 hours,36 hours, 48 hours, 60 hours or 72 hour) to remove carboxyl groups therefrom, thereby achieving a further anti-calcification treatment.

At last, a fourth washing process may be carried out with the same parameters as the second washing process.

Step 5 includes preparing a reducing solution; and subjecting the biological tissue material to a third anti-calcification treatment using the reducing solution for protecting free aldehyde groups.

In step 5, specifically, at first, the reducing solution may be prepared with a concentration of 0.01%-50% (in particular, for example, 0.01%, 1%, 10%, 20%, 30%, 40% or 50%). The reducing solution is a solution of at least one of metaphosphate, an amino acid, 3-dimethylaminopropyl and amino alcohol and has a pH value of 5.0-9.0 (in particular, for example, 5.0, 5.5, 6.0, 6.5, 7.0, 7.5, 8.0, 8.5 or 9.0).

Subsequently, the biological tissue material may be placed in the reducing solution at a temperature of 0-50 °C (in particular, for example, 0 °C, 10 °C, 20 °C, 30 °C, 40 °C or 50 °C) for 1-72 hours (in particular, for example, 1 hour, 12 hours, 24 hours, 36 hours, 48 hours, 60 hours or 72 hours) in order to protect free aldehyde groups on the surface of the biological tissue material against subsequent oxidation into carboxyl groups capable of binding with calcium ions. In this way, the risk of calcification of the biological tissue material can be additionally reduced.

At last, a fifth washing process may be carried out with the same parameters as the second washing process.

In particular, step 6 includes preparing dehydrating solutions including first to M-th dehydrating solutions with gradually increasing concentrations, and subjecting the biological tissue material to a dehydration treatment using the dehydrating solutions, wherein the concentration of the M-th dehydrating solution is lower than the concentration of the N-th decellularization solution, and M is a positive integer that is greater than or equal to 2.

Specifically, at first, the dehydrating solutions may be prepared. The dehydrating solutions are solutions of one or more of acetone, ethanol, n-butanol and tert-butanol. The dehydrating solutions are prepared so that they include first to M-th dehydrating solutions with gradually increasing concentrations. Moreover, the concentration of the M-th dehydrating solution is lower than the concentration of the N-th decellularization solution in order to ensure structural integrity of collagen in the biological tissue material. M is a positive integer that is greater than or equal to 2.

Next, the biological tissue material may be successively placed in the first to M-th dehydrating solutions at a temperature of 0-50 °C (in particular, for example, 0 °C, 10 °C, 20 °C, 30 °C, 40 °C or 50 °C) for 1-120 hours (in particular, for example, 1 hour, 12 hours, 24 hours, 48 hours, 72 hours, 96 hours or 120 hours). In this way, the dried (or dry) biological tissue material can be obtained.

After step 6, the method may further include anaerobic packaging and sterilization of the dry biological tissue material. The sterilization may be accomplished by ethylene oxide (EO) or by radiation, with radiation sterilization being more preferred. The anaerobic packaging can prevent oxidation of aldehyde groups remaining in the dry biological tissue material during the packing and sterilization processes, thereby further reducing the risk of calcification of the biological tissue material.

According to embodiments of the present invention, the dry biological tissue material is prepared using a method as defined above.

Dry biological tissue materials provided in embodiments of present invention may be used to produce dry valves.

Objects, advantages and features of the present invention will become more apparent from the following more detailed description of the examples and comparative examples below. It is to be noted that these examples given in the exemplary context of bovine pericardia being used are merely some optional but not all possible examples of the invention. Accordingly, the following examples shall not be construed as limiting the present invention in any way.

### <Reference embodiment - Example 1>

In step 1, bovine pericardia were obtained from a local slaughterhouse. After unwanted portions thereof were stripped away, desired portions were selected from the remainder and washed. After that, they were fixed for 14 days in a glutaraldehyde solution with a concentration of 0.625% prepared in advance. The fixed bovine pericardium portions were cut into 50 mm×50 mm bovine pericardium samples with a cutter (e.g., Speed x-100 from Trotec). The samples were washed with a saline solution (e.g., from Huaren Pharmaceutical) at room temperature 2-3 times, 10 minutes each time.

In step 2, three aqueous ethanol solutions with different ethanol concentrations of 50%, 75% and 90% were prepared, and the bovine pericardium samples were successively soaked in the aqueous ethanol solutions in order from the lowest concentration to the highest concentration at room temperature for 24 hours at each concentration. After that, the bovine pericardium samples were submerged in a mixed solution of 10 mM Tris-HCl and 1 mM EDTA, and the pH value was adjusted to 7.4±0.1 and the temperature to 5±1 °C. Five hours later, they were again submerged in a 1% SDS solution at room temperature for 5 hours. Finally, they were washed with a saline solution 3 times, 5 minutes each time.

In step 3, an aqueous ethanol solution with an ethanol concentration of 70% and a pH value of 7.3-7.5 was prepared as a dissolving solution, and the bovine pericardium samples were soaked in the dissolving solution at room temperature for 48 hours and then washed with a saline solution for 3 times, 5 minutes each time.

In step 4, 10g of sodium carbonate was weighed and dissolved in 1L of a PBS solution in a container, and the pH value of the solution was adjusted to 7.3-7.5. In this way, a removal solution was obtained. The bovine pericardium samples were soaked in the removal solution at room temperature for 24 hours and then washed with a saline solution for 3 times, 5 minutes each time.

In step 5, a protective (or reducing) solution containing 1% of amino alcohol was prepared and its pH value was adjusted to 7.3-7.5. The bovine pericardium samples were submerged in the protective solution at room temperature for 48 hours and then washed with a saline solution for 3 times, 5 minutes each time.

In step 6, two glycerol solutions with different glycerol concentrations of 60% and 80% were prepared as dehydrating solutions. The bovine pericardium samples were soaked first in the dehydrating solution containing 60% of glycerol at 37 °C for 48 hours, and then in the dehydrating solution containing 80% of glycerol at 37 °C for 48 hours.

In step 7, the bovine pericardium samples were vacuum packed in aluminum foil bags using a vacuum packing machine (e.g., J-VB06, Jushida Intelligent Packaging Equipment), and then sterilized with gamma ray radiation.

### <Comparative Example 1>

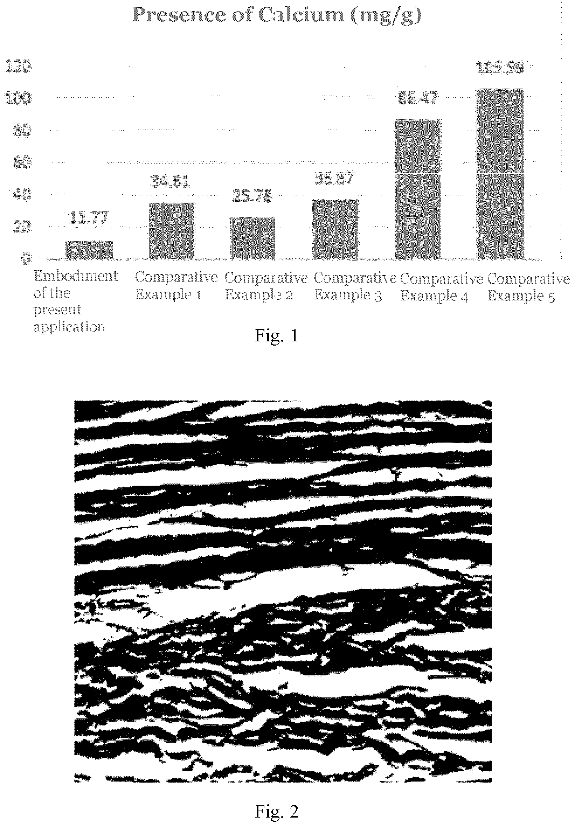

Dry biological tissue material samples were prepared using a method similar to that of Example 1 except that step 3 was omitted, and presence of calcium therein was tested to test anti-calcification performance, as shown in Fig. 1.

### <Comparative Example 2>

Dry biological tissue material samples were prepared using a method similar to that of Example 1 except that step 4 was omitted, and presence of calcium therein was tested as to test anti-calcification performance, as shown in Fig. 1.

### <Comparative Example 3>

Dry biological tissue material samples were prepared using a method similar to that of Example 1 except that step 5 was omitted, and presence of calcium therein was tested to test anti-calcification performance, as shown in Fig. 1.

### <Comparative Example 4>

Dry biological tissue material samples were prepared using a method similar to that of Example 1 except that steps 3-5 was omitted, and presence of calcium therein was tested to test anti-calcification performance, as shown in Fig. 1.

### <Comparative Example 5>

Dry biological tissue material samples were prepared using a method similar to that of Example 1 except that steps 3-5 were omitted and step 6 was replaced with step 6' in which dehydrated bovine pericardium samples was placed and sealed in paper-plastic bags and subjected to EO sterilization. Presence of calcium in the samples was tested to test anti-calcification performance, as shown in Fig. 1.

### <Comparative Example 6>

Dry biological tissue material samples were prepared using a method similar to that of Example 1 except that step 2 was omitted, and presence of calcium therein was tested to test anti-calcification performance, as shown in Table 1.

### <Comparative Example 7>

Dry biological tissue material samples were prepared using a method similar to that of Example 1 except that step 6 was replaced with step 6", wherein the step 6" includes: preparing two glycerol solutions with different glycerol concentrations of 70% and 95% as dehydrating solutions; and soaking bovine pericardium samples first in the 70% glycerol solution at 37 °C for 48 hours; and then in the 95% glycerol solution at 37 °C for 48 hours. Presence of calcium in the samples was tested to test anti-calcification performance, as shown in Table 1.

As can be seen from Fig. 1, the bovine pericardium samples obtained from the method according to the reference embodiment and methods according to Comparative Examples 1 to 5 were subcutaneously implanted in rats, taken out 8 weeks later and measured on an atomic absorption spectrophotometer for presence of calcium therein, the results show that the bovine pericardium samples obtained from the method according to the reference embodiment exhibit the lowest presence of calcium. Therefore, the methods according to the claims of the present application can significantly improve anti-calcification performance of bovine pericardium samples.

As can be seen from Table 1 below, among the dry bovine pericardium samples obtained from the methods according to the reference embodiment and Comparative Examples 6 and 7, sample obtained from the method according to the reference embodiment contains less water and possess excellent mechanical properties. Moreover, the method according to the present application can impart anti-immune properties.

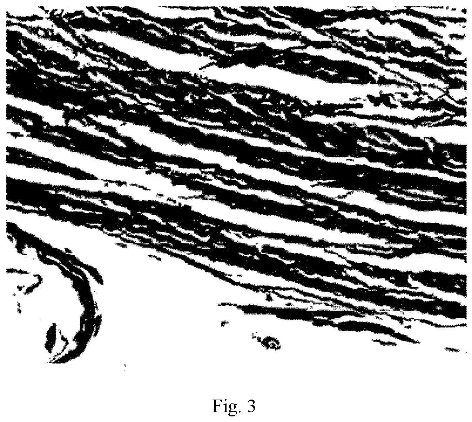

Images of Figs. 2 to 3 of HE-stained bovine pericardium samples prepared respectively using the methods according to the reference embodiment and Comparative Example 7 show that the bovine pericardium sample obtained from the method of Comparative Example 7 has structurally destructed collagen, while the bovine pericardium sample obtained from the method according to ] the has consistently oriented collagen fiber, indicating no reference embodiment significant destruction.

**Table 1**

| | DNA Residue (ug/mL) | Water Content (%) | Tensile Strength at Breakage (N) |
|---|---|---|---|
| Reference embodiment | 0.143±0.019 | 25.3±0.7 | 18.1±3.1 |
| Comparative Example 6 | 0.953±0.013 | 27.6±2.2 | 19.4±2.5 |
| Comparative Example 7 | 0.164±0.028 | 21.9±3.1 | 17.8±1.5 |

## Claims

1. A preparation method for a dry biological tissue material, comprising the steps of:
(a) obtaining a biological tissue material that has undergone a cross-linking treatment using a cross-linking agent, wherein the cross-linking agent is an aldehyde-based cross-linking agent, and the aldehyde-based cross-linking agent comprises glutaraldehyde, formaldehyde and glyoxal;
(b) performing a decellularization treatment on the biological tissue material using decellularization solutions which are aqueous solutions of a monoalcohol or a polyol, wherein the decellularization solutions include first to N-th decellularization solutions with gradually increasing concentrations, and wherein N is a positive integer that is greater than or equal to 2,
washing the biological tissue material that has undergone decellularization treatment with a mixed solution of 1-10 mM Tris-HCl with a pH value of 6.8-8.6 and 1-5 mM EDTA at a temperature of 0-10 °C for 1-24 hours, and then further treating the biological tissue material with an SDS solution with a concentration of 0.5-2% for 1-24 hours;
(c) performing a first anti-calcification treatment on the biological tissue material using a dissolving solution, wherein the dissolving solution comprises at least one of methanol, ethanol, chloroform and diethyl ether and has a pH value of 5.0-9.0, and wherein the dissolving solution is used to dissolve phospholipid impurities in the biological tissue;
(d) performing a second anti-calcification treatment on the biological tissue material using a removal solution, wherein the removal solution comprises at least one of sodium carbonate, sodium bicarbonate, 1,1,2-trimethyl-propyl-borane, diisobutylaluminum hydride, borane and sodium borohydride and has a pH value of 5.0-9.0, and wherein the removal solution is used to remove carboxyl groups produced in the cross-linking and decellularization treatments as a result of oxidation of aldehyde groups;
(e) performing a third anti-calcification treatment on the biological tissue material using a reducing solution, wherein the reducing solution comprises a solution of at least one of metaphosphate, amino acid, 3-dimethylaminopropyl and amino alcohol and has a pH value of 5.0-9.0, and wherein the reducing solution is used to protect free aldehyde groups; and
(f) performing a dehydration treatment on the biological tissue material using dehydrating solutions to obtain the dry biological tissue material, wherein the dehydrating solutions comprise a solution of one of acetone, ethanol, n-butanol and tert-butanol, or a solution of a mixture thereof, wherein the dehydrating solutions include first to M-th dehydrating solutions with gradually increasing concentrations, and wherein the concentration of the M-th dehydrating solution is lower than the concentration of the N-th decellularization solution, and M is a positive integer that is greater than or equal to 2.

2. The preparation method of claim 1, wherein step (b) further comprises:
(a) preparing the decellularization solutions; and
(b) sequentially placing the biological tissue material in the first to N-th decellularization solutions for at least 1 hour in each solution, so as to perform the decellularization treatment on the biological tissue material.

3. The preparation method of claim 2, wherein sequentially placing the biological tissue material in the first to N-th decellularization solutions for at least 1 hour in each solution comprises:
(a) when the decellularization solutions include: a first decellularization solution with a concentration of 50-70%; and a second decellularization solution with a concentration of 70-100%, placing the biological tissue material in the first decellularization solution for at least 1 hour and then in the second decellularization solution for at least 1 hour; or
(b) when the decellularization solutions include: a first decellularization solution with a concentration of 50-60%; a second decellularization solution with a concentration of 60-80%; and a third decellularization solution with a concentration of 80-100%, placing the biological tissue material in the first decellularization solution for at least 1 hour, then in the second decellularization solution for at least 1 hour and finally in the third decellularization solution for at least 1 hour.

4. The preparation method of claim 1, wherein step (c) specifically comprises:
(a) preparing the dissolving solution with a concentration of 1-100%;
(b) placing the biological tissue material in the dissolving solution at 0°C -50 °C for 1-72 hours, so as to perform the first anti-calcification treatment on the biological tissue material; and
(c) washing the biological tissue material that has undergone the first anti-calcification treatment.

5. The preparation method of claim 1, wherein step (d) specifically comprises:
(a) preparing the removal solution with a concentration of 0.01-50%;
(b) placing the biological tissue material in the removal solution at -100°C -50 °C for 1-72 hours, so as to perform the second anti-calcification treatment; and
(c) washing the biological tissue material that has undergone the second anti-calcification treatment.

6. The preparation method of claim 1, wherein step (e) specifically comprises:
(a) preparing the reducing solution with a concentration of 0.01-50%;
(b) placing the biological tissue material in the reducing solution at 0°C -50 °C for 1-72 hours, so as to perform the third anti-calcification treatment on the biological tissue material; and
(c) washing the biological tissue material that has undergone the third anti-calcification treatment.

7. The preparation method of claim 1, wherein step (f) specifically comprises:
(a) preparing the dehydrating solutions; and
(b) sequentially placing the biological tissue material in the first to M-th dehydrating solutions at 0°C-50 °C for 1-120 hours in each solution, so as to perform the dehydration treatment on the biological tissue material.

8. The preparation method of claim 1, further comprising after the step (f):
anaerobically packaging and sterilizing the dry biological tissue material, wherein the sterilization is accomplished by ethylene oxide (EO) or by radiation.

9. A dry biological tissue material prepared by the preparation method as defined in any one of claims 1 to 8.

10. The dry biological tissue material of claim 9, wherein the dry biological tissue material is used to produce a dry valve.

## Patentansprüche

1. Herstellungsverfahren für ein trockenes biologisches Gewebematerial, das die Schritte umfasst zum:
(a) Erhalten eines biologischen Gewebematerials, das einer Vernetzungsbehandlung unter Verwendung eines Vernetzungsmittels unterzogen wurde, wobei das Vernetzungsmittel ein Vernetzungsmittel auf Aldehydbasis ist und das Vernetzungsmittel auf Aldehydbasis Glutaraldehyd, Formaldehyd und Glyoxal umfasst;
(b) Durchführen einer Dezellularisierungsbehandlung an dem biologischen Gewebematerial unter Verwendung von Dezellularisierungslösungen, die wässrige Lösungen eines Monoalkohols oder eines Polyols sind, wobei die Dezellularisierungslösungen erste bis N-te Dezellularisierungslösungen mit allmählich ansteigenden Konzentrationen beinhalten und wobei N eine positive ganze Zahl größer oder gleich 2 ist,
Waschen des biologischen Gewebematerials, das einer Dezellularisierungsbehandlung unterzogen wurde, mit einer Mischlösung aus 1-10 mM Tris-HCl mit einem pH-Wert von 6,8-8,6 und 1-5 mM EDTA bei einer Temperatur von 0-10°C 1-24 Stunden lang und danach Weiterbehandeln des biologischen Gewebematerials mit einer SDS-Lösung mit einer Konzentration von 0,5-2 % 1-24 Stunden lang;
(c) Durchführen einer ersten Antikalzifizierungsbehandlung an dem biologischen Gewebematerial unter Verwendung einer lösenden Lösung, wobei die lösende Lösung mindestens eines von Methanol, Ethanol, Chloroform und Diethylether umfasst und einen pH-Wert von 5,0-9,0 aufweist und wobei die lösende Lösung verwendet wird, um Phospholipidverunreinigungen im biologischen Gewebe zu lösen;
(d) Durchführen einer zweiten Antikalzifizierungsbehandlung an dem biologischen Gewebematerial unter Verwendung einer Entfernungslösung, wobei die Entfernungslösung mindestens eines von Natriumcarbonat, Natriumbicarbonat, 1,1,2-Trimethylpropylboran, Diisobutylaluminiumhydrid, Boran und Natriumborhydrid umfasst und einen pH-Wert von 5,0-9,0 aufweist, und wobei die Entfernungslösung verwendet wird, um Carboxylgruppen zu entfernen, die bei den Vernetzungs- und Dezellularisierungsbehandlungen als Ergebnis von Oxidation von Aldehydgruppen entstanden sind;
(e) Durchführen einer dritten Antikalzifizierungsbehandlung an dem biologischen Gewebematerial unter Verwendung einer Reduktionslösung, wobei die Reduktionslösung eine Lösung aus mindestens einem von Metaphosphat, Aminosäure, 3-Dimethylaminopropyl und Aminoalkohol umfasst und einen pH-Wert von 5,0-9,0 aufweist, und wobei die Reduktionslösung verwendet wird, um freie Aldehydgruppen zu schützen; und
(f) Durchführen einer Dehydrierungsbehandlung an dem biologischen Gewebematerial unter Verwendung von Dehydrierungslösungen, um das trockene biologische Gewebematerial zu erhalten, wobei die Dehydrierungslösungen eine Lösung aus einem von Aceton, Ethanol, n-Butanol und tert-Butanol oder eine Lösung aus einem Gemisch davon umfassen, wobei die Dehydrierungslösungen erste bis M-te Dehydrierungslösungen mit allmählich ansteigenden Konzentrationen beinhalten und wobei die Konzentration der M-ten Dehydrierungslösung niedriger ist als die Konzentration der N-ten Dezellularisierungslösung und M eine positive ganze Zahl größer oder gleich 2 ist.

2. Herstellungsverfahren nach Anspruch 1, wobei Schritt (b) weiter umfasst:
(a) Herstellen der Dezellularisierungslösungen; und
(b) nacheinander Platzieren des biologischen Gewebematerials in die erste bis N-te Dezellularisierungslösung, mindestens eine Stunde lang in jeder Lösung, um die Dezellularisierungsbehandlung am biologischen Gewebematerial durchzuführen.

3. Herstellungsverfahren nach Anspruch 2, wobei nacheinander Platzieren des biologischen Gewebematerials in die erste bis N-te Dezellularisierungslösung, mindestens 1 Stunde lang in jeder Lösung umfasst:
(a) wenn die Dezellularisierungslösungen beinhalten: eine erste Dezellularisierungslösung mit einer Konzentration von 50-70%; und eine zweite Dezellularisierungslösung mit einer Konzentration von 70-100%, Platzieren des biologischen Gewebematerials in die erste Dezellularisierungslösung, mindestens 1 Stunde lang, und danach in die zweite Dezellularisierungslösung, mindestens 1 Stunde lang; oder
(b) wenn die Dezellularisierungslösungen beinhalten: eine erste Dezellularisierungslösung mit einer Konzentration von 50-60%; eine zweite Dezellularisierungslösung mit einer Konzentration von 60-80%; und eine dritte Dezellularisierungslösung mit einer Konzentration von 80-100%, Platzieren des biologischen Gewebematerials in die erste Dezellularisierungslösung, mindestens 1 Stunde lang, danach in die zweite Dezellularisierungslösung, mindestens 1 Stunde lang, und schließlich in die dritte Dezellularisierungslösung, mindestens 1 Stunde lang.

4. Herstellungsverfahren nach Anspruch 1, wobei Schritt (c) speziell umfasst:
(a) Herstellen der lösenden Lösung mit einer Konzentration von 1-100%;
(b) Platzieren des biologischen Gewebematerials in die Lösung bei 0°C-50°C 1-72 Stunden lang, um die erste Antikalzifizierungsbehandlung am biologischen Gewebematerial durchzuführen; und
(c) Waschen des biologischen Gewebematerials, das der ersten Antikalzifizierungsbehandlung unterzogen wurde.

5. Herstellungsverfahren nach Anspruch 1, wobei Schritt (d) speziell umfasst:
(a) Herstellen der Entfernungslösung mit einer Konzentration von 0,01-50%;
(b) Platzieren des biologischen Gewebematerials in die Entfernungslösung bei -100°C -50°C 1 bis 72 Stunden lang, um die zweite Antikalzifizierungsbehandlung durchzuführen; und
(c) Waschen des biologischen Gewebematerials, das der zweiten Antikalzifizierungsbehandlung unterzogen wurde.

6. Herstellungsverfahren nach Anspruch 1, wobei Schritt (e) speziell umfasst:
(a) Herstellen der Reduktionslösung mit einer Konzentration von 0,01-50%;
(b) Platzieren des biologischen Gewebematerials in die Reduktionslösung bei 0°C-50°C 1-72 Stunden lang, um die dritte Antikalzifizierungsbehandlung an dem biologischen Gewebematerial durchzuführen; und
(c) Waschen des biologischen Gewebematerials, das der dritten Antikalzifizierungsbehandlung unterzogen wurde.

7. Herstellungsverfahren nach Anspruch 1, wobei Schritt (f) speziell umfasst:
(a) Herstellen der Dehydrierungslösungen; und
(b) nacheinander Platzieren des biologischen Gewebematerials in die erste bis M-te Dehydrierungslösung bei 0°C-50°C 1-120 Stunden lang in jeder Lösung, um die Dehydrierungsbehandlung am biologischen Gewebematerial durchzuführen.

8. Herstellungsverfahren nach Anspruch 1, das weiter nach dem Schritt (f) umfasst:
anaerobisch Verpacken und Sterilisieren des trockenen biologischen Gewebematerials, wobei die Sterilisierung durch Ethylenoxid (EO) oder durch Bestrahlung erfolgt.

9. Trockenes biologisches Gewebematerial, das durch das Herstellungsverfahren nach einem der Ansprüche 1 bis 8 hergestellt wird.

10. Trockenes biologisches Gewebematerial nach Anspruch 9, wobei das trockene biologische Gewebematerial verwendet wird, um eine trockene Klappe zu produzieren.

## Revendications

1. Procédé de préparation d'un matériau tissulaire biologique sec, comprenant les étapes consistant à :
(a) obtenir un matériau tissulaire biologique qui a subi un traitement de réticulation à l'aide d'un agent de réticulation, dans lequel l'agent de réticulation est un agent de réticulation à base d'aldéhyde, et l'agent de réticulation à base d'aldéhyde comprend du glutaraldéhyde, du formaldéhyde et du glyoxal ;
(b) effectuer un traitement de décellularisation sur le matériau tissulaire biologique à l'aide de solutions de décellularisation qui sont des solutions aqueuses d'un monoalcool ou d'un polyol, dans lequel les solutions de décellularisation incluent des première à N-ième solutions de décellularisation avec des concentrations croissant graduellement, et dans lequel N est un entier positif qui est supérieur ou égal à 2,
laver le matériau tissulaire biologique qui a subi un traitement de décellularisation avec une solution mixte de 1 à 10 mM de Tris-HCl avec une valeur de pH de 6,8 à 8,6 et de 1 à 5 mM d'EDTA à une température de 0 à 10°C pendant 1 à 24 heures, puis traiter à nouveau le matériau tissulaire biologique avec une solution de SDS avec une concentration de 0,5 à 2 % pendant 1 à 24 heures ;
(c) effectuer un premier traitement d'anti-calcification sur le matériau tissulaire biologique en utilisant une solution de dissolution, dans lequel la solution de dissolution comprend au moins un parmi méthanol, éthanol, chloroforme et éther diéthylique, et présente une valeur de pH de 5,0 à 9,0, et dans laquelle la solution de dissolution est utilisée pour dissoudre les impuretés phospholipidiques dans le tissu biologique ;
(d) effectuer un deuxième traitement anti-calcification sur le matériau tissulaire biologique en utilisant une solution d'élimination, dans lequel la solution d'élimination comprend au moins un parmi carbonate de sodium, bicarbonate de sodium, 1,1,2-triméthyl-propyl-borane, hydrure de diisobutylaluminium, borane et borohydrure de sodium, et présente une valeur de pH de 5,0 à 9,0, et dans lequel la solution d'élimination est utilisée pour éliminer des groupes carboxyle produits dans les traitements de réticulation et de décellularisation à la suite d'une oxydation de groupes aldéhydes ;
(e) effectuer un troisième traitement anti-calcification sur le tissu biologique en utilisant une solution de réduction, dans lequel la solution de réduction comprend une solution d'au moins un parmi métaphosphate, acide aminé, 3-diméthylaminopropyle et aminoalcool, et présentant un pH de 5,0 à 9,0, et dans lequel la solution de réduction est utilisée pour protéger des groupes aldéhyde libres ; et
(f) effectuer un traitement de déshydratation sur le matériau tissulaire biologique en utilisant des solutions de déshydratation pour obtenir le matériau de tissu biologique sec, dans lequel les solutions de déshydratation comprennent une solution d'un parmi acétone, éthanol, n-butanol et tert-butanol, ou une solution d'un mélange de ceux-ci, dans lequel les solutions de déshydratation incluent des première à M-ième solutions de déshydratation avec des concentrations croissant graduellement, et dans lequel la concentration de la M-ième solution de déshydratation est inférieure à la concentration de la N-ième solution de décellularisation, et M est un entier positif qui est supérieur ou égal à 2.

2. Procédé de préparation selon la revendication 1, dans lequel l'étape (b) comprend en outre :
(a) la préparation des solutions de décellularisation ; et
(b) le placer séquentiel du matériau tissulaire biologique dans les première à N-ième solutions de décellularisation pendant au moins 1 heure dans chaque solution, de manière à effectuer le traitement de décellularisation sur le matériau tissulaire biologique.

3. Procédé de préparation selon la revendication 2, dans lequel le placement séquentiel du matériau tissulaire biologique dans les première à N-ième solutions de décellularisation pendant au moins 1 heure dans chaque solution comprend :
(a) lorsque les solutions de décellularisation incluent : une première solution de décellularisation avec une concentration de 50 à 70 % ; et une deuxième solution de décellularisation avec une concentration de 70 à 100 %, le placement du matériau tissulaire biologique dans la première solution de décellularisation pendant au moins 1 heure, puis dans la deuxième solution de décellularisation pendant au moins 1 heure ; ou
(b) lorsque les solutions de décellularisation incluent : une première solution de décellularisation avec une concentration de 50 à 60 % ; une deuxième solution de décellularisation avec une concentration de 60 à 80 % ; et une troisième solution de décellularisation avec une concentration de 80 à 100 %, le placement du matériel tissulaire biologique dans la première solution de décellularisation pendant au moins 1 heure, puis dans la deuxième solution de décellularisation pendant au moins 1 heure et enfin dans la troisième solution de décellularisation pendant au moins 1 heure.

4. Procédé de préparation selon la revendication 1, dans lequel l'étape (c) comprend spécifiquement :
(a) la préparation de la solution de dissolution avec une concentration de 1 à 100 % ;
(b) le placement du matériau tissulaire biologique dans la solution de dissolution entre 0°C et 50°C pendant 1 à 72 heures, de manière à effectuer le premier traitement anti-calcification sur le matériau tissulaire biologique ; et
(c) le lavage du matériau tissulaire biologique qui a subi le premier traitement anti-calcification.

5. Procédé de préparation selon la revendication 1, dans lequel l'étape (d) comprend spécifiquement :
(a) la préparation de la solution d'élimination avec une concentration de 0,01 à 50 % ;
(b) le placement du matériau tissulaire biologique dans la solution d'élimination entre -100°C et 50°C pendant 1 à 72 heures, de manière à effectuer le deuxième traitement anti-calcification ; et
(c) le lavage du matériau tissulaire biologique qui a subi le deuxième traitement anti-calcification.

6. Procédé de préparation selon la revendication 1, dans lequel l'étape (e) comprend spécifiquement :
(a) la préparation de la solution de réduction avec une concentration de 0,01 à 50 % ;
(b) le placement du matériau tissulaire biologique dans la solution de réduction entre 0°C et 50°C pendant 1 à 72 heures, de manière à effectuer le troisième traitement anti-calcification sur le matériau tissulaire biologique ; et
(c) le lavage du matériau tissulaire biologique qui a subi le troisième traitement anti-calcification.

7. Procédé de préparation selon la revendication 1, dans lequel l'étape (f) comprend spécifiquement :
(a) la préparation des solutions de déshydratation ; et
(b) le placement séquentiel du matériau tissulaire biologique dans les première à M-ième solutions de déshydratation entre 0°C et 50°C pendant 1 à 120 heures dans chaque solution, de manière à effectuer le traitement de déshydratation sur le matériau tissulaire biologique.

8. Procédé de préparation selon la revendication 1, comprenant en outre, après l'étape (f) :
un conditionnement et une stérilisation anaérobies du matériau tissulaire biologique sec, dans lequel la stérilisation est réalisée par l'intermédiaire d'oxyde d'éthylène (OE) ou par rayonnement.

9. Matériau tissulaire biologique sec préparé par le procédé de préparation tel que défini dans l'une quelconque des revendications 1 à 8.

10. Matériau tissulaire biologique sec selon la revendication 9, dans lequel le matériau tissulaire biologique sec est utilisé pour produire une valve sèche.
